# DEMANDE DE BREVET EUROPEEN

(11) **EP 1 457 193 A1**
(43) Date de publication de la demande: **15.09.2004**
(21) Numéro de dépôt: 04290592.7
(22) Date de dépôt: 04.03.2004
(51) Int. Cl.: A61K 7/025

(54) **Composition cosmétique contenant un polyester de triglycéride d'acides carboxyliques hydroxyles et un composé pâteux**

(30) Priorité: 12.03.2003 FR 0303077; 12.03.2003 FR 0303079
(71) Demandeur: L'OREAL, 75008 Paris (FR)
(72) Inventeur: Blin, Xavier, 75015 Paris (FR); Filippi, Vanina, 75015 Paris (FR)
(74) Mandataire: Boulard, Denis

(57) **Abrégé**

L'invention se rapporte à une composition cosmétique de soin ou de maquillage contenant a) au moins un polyester résultant de l'estérification d'au moins un triglycéride d'acide(s) carboxylique(s) hydroxylé(s) par un acide monocarboxylique aliphatique et par un acide dicarboxylique aliphatique, et b) au moins un composé pâteux.

Cette composition permet d'obtenir un dépôt sur les matières kératiniques glissant, brillant, confortable, net aux contours, qui ne migre pas, dont l'intensité de la couleur est améliorée et/ou dont la tenue de la couleur après épreuves est améliorée.

## Description

La présente invention se rapporte à une composition cosmétique de maquillage ou de soin de la peau, y compris du cuir chevelu, aussi bien du visage que du corps humain, des lèvres ou des phanères des êtres humains, comme les cheveux, les cils, les sourcils ou les ongles, comprenant un milieu cosmétiquement acceptable contenant un polyester particulier.

Cette composition contient au moins un polyester résultant de l'estérification d'au moins un triglycéride d'acide(s) carboxylique(s) hydroxylé(s) par un acide monocarboxylique aliphatique et par un acide dicarboxylique aliphatique.

Cette composition possède des propriétés cosmétiques remarquables et confère en particulier au maquillage ou au soin des propriétés de brillance, de glissant à l'application, de confort, de tenue de la couleur dans le temps et après épreuves, de tenue de la brillance dans le temps, de non-migration, de netteté des contours et/ou d'intensité de la couleur.

La composition de l'invention peut en particulier constituer un produit de maquillage du corps, des lèvres ou des phanères d'êtres humains ayant en particulier des propriétés de soin et/ou de traitement non thérapeutique. Elle constitue notamment un rouge à lèvres ou un brillant à lèvres, un fard à joues ou à paupières, un produit pour tatouage, un mascara, un eye-liner, un vernis à ongles, un produit de bronzage artificiel de la peau, un produit de coloration ou de soin des cheveux.

De façon surprenante, les inventeurs ont trouvé qu'il était possible d'obtenir une composition contenant un polyester résultant de l'estérification d'au moins un triglycéride d'acide(s) carboxylique(s) hydroxylé(s) par un acide monocarboxylique aliphatique et par un acide dicarboxylique aliphatique, qui est brillante, confortable et qui ne migre pas. En outre, l'intensité de la couleur de la composition est bien meilleure que celle des compositions de l'art antérieur.

Cette composition présente en outre une bonne dispersion des pigments et/ou des charges présents dans la composition, elle n'exsude pas lorsqu'elle est sous forme de stick, elle présente de bonnes propriétés d'étalement et de glissant et confère, en outre, au film déposé des contours nets, des propriétés de bonne tenue de la brillance et de la couleur dans le temps (non virage de couleur pendant au moins 3 heures, disparition du maquillage de façon homogène). Elle est, en outre, stable notamment plusieurs mois à température ambiante (25°C pendant plus d'un an), mais aussi à la chaleur (47°C pendant 2 mois) et aux ultraviolets, sans dégradation de l'odeur au cours du temps.

De façon plus précise, l'invention a pour objet une composition contenant a) un polyester résultant de l'estérification, d'au moins un triglycéride d'acide(s) carboxylique(s) hydroxylé(s), par un acide monocarboxylique aliphatique et par un acide dicarboxylique aliphatique, et b) au moins un composé pâteux.

### Polyester de triglycéride d'acide(s) aliphatique(s) hydroxylé(s)

La composition comprend au moins un polyester résultant de l'estérification d'au moins un triglycéride d'acide(s) carboxylique(s) hydroxylé(s) par un acide monocarboxylique aliphatique et par un acide dicarboxylique aliphatique, éventuellement insaturé.

Par acide carboxylique hydroxylé, on entend un acide hydroxy-carboxylique aliphatique. Par triglycéride d'acide(s) carboxylique(s) hydroxylé(s), on entend un glycérol substitué par trois restes d'acides carboxyliques hydroxylés, lesquelles peuvent être identiques ou différents. Par exemple, un triglycéride obtenu par réaction d'un équivalent de glycérol et trois équivalents d'un acide carboxylique hydroxylé sera dénommé "triglycéride d'acide carboxylique hydroxylé". Un triglycéride obtenu par réaction d'un équivalent de glycérol avec trois équivalents d'un mélange d'au moins deux acides carboxyliques hydroxylés différents sera dénommé "triglycéride d'acide(s) carboxylique(s) hydroxylé(s)".

Le polyester selon l'invention est de préférence liquide à température ambiante (comprise généralement entre 20 et 25°C) et pression atmosphérique (760 mm de Hg).

De préférence, le polyester selon l'invention présente une viscosité à 25°C supérieure à 500 cP (50 Pa.s), de préférence allant de 900 à 10 000 cP (90 à 1 000 Pa.s) et mieux de 950 à 5 000 cP (95 à 500 Pa.s), mesurée en particulier avec un viscosimètre type Brookfield RV ou un viscosimètre Brookfield "DV-II+" de type LV équipé d'une aiguille n°1 tournant entre 0,5 et 10 tr/min. La mesure de la viscosité est prise lorsque la valeur de la mesure est stabilisée, en général au bout de 10 minutes.

De préférence, le polyester selon l'invention a un indice de réfraction supérieur ou égal à 1,47 et notamment allant de 1,47 à 1,55 (l'indice de réfraction étant défini pour la raie D du sodium).

Selon un mode de mise en oeuvre, le polyester est avantageusement obtenu par deux réactions d'estérification d'un triglycéride d'acide(s) carboxylique(s) hydroxylé(s) : une estérification par un acide monocarboxylique aliphatique et une estérification par un acide dicarboxylique aliphatique.

Dans ce mode de mise en oeuvre, le polyester est avantageusement obtenu par
a) l'estérification par un acide monocarboxylique aliphatique d'une partie des fonctions hydroxyles d'un triglycéride d'acide(s) carboxylique(s) hydroxylé(s), et par
b) l'estérification par un acide dicarboxylique aliphatique des fonctions hydroxyles restantes dudit triglycéride d'acide(s) carboxylique(s) hydroxylé(s) estérifié par ledit acide monocarboxylique aliphatique.
L'estérification par un acide monocarboxylique est de préférence conduite avant l'estérification par un acide dicarboxylique aliphatique.

Le ou les acides carboxyliques hydroxylés (précurseurs) du triglycéride d'acide(s) carboxylique(s) hydroxylé(s)) sont de préférence choisis parmi les acides carboxyliques aliphatiques hydroxylés comprenant de 6 à 40 atomes de carbone, de préférence de 10 à 34 atomes de carbone et mieux de 12 à 28 atomes de carbone, de préférence de 16 à 20 atomes de carbone, de préférence de 18 atomes de carbone.

Le ou les acides carboxyliques hydroxylés sont de préférence choisis parmi les acides gras saturés ou insaturés.

Le ou les acides carboxyliques hydroxylés peuvent être choisis parmi :
i) les acides monocarboxyliques aliphatiques mono hydroxylés linéaires saturés de formule :
   (1) 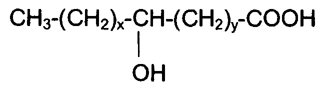 avec 3 ≤ x + y ≤ 37
   ou (2) HO-CH₂-(CH₂)ₓ-COOH avec 4 ≤ x ≤ 38 ;
ii) les acides monocarboxyliques aliphatiques mono hydroxylés ramifiés saturés de formule :
   (3) 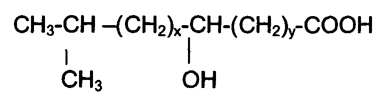 avec 1 ≤ x + y ≤ 35 Ou (3') l'acide 2-éthyl 3-hydroxy caprylique de formule : 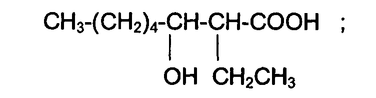
iii) les acides monocarboxyliques aliphatiques mono hydroxylés insaturés de formule :
   (4) 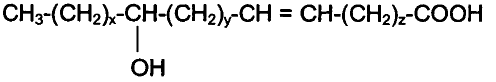 avec 1 ≤ x + y + z ≤ 35
   ou (5) 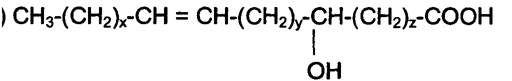 avec 1 ≤ x + y + z ≤ 35
   ou (6) HOCH₂-(CH₂)ₓ-CH = CH-(CH₂)_{y}-COOH avec 2 ≤ x + y ≤ 36 ;
iv) les acides monocarboxyliques aliphatiques poly hydroxylés saturés de formule :
   (7) 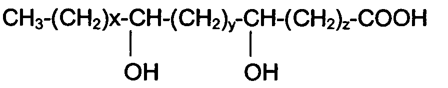 avec 2 ≤ x + y + z ≤ 36; et les acides monocarboxyliques aliphatiques poly hydroxylés insaturés correspondants,
v) les polyacides aliphatiques mono hydroxylés saturés de formule :
   (8) 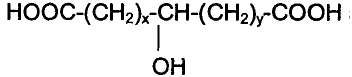 avec 3 ≤ x + y ≤ 37 ; et les polyacides aliphatiques mono hydroxylés insaturés correspondants,
vi) les polyacides aliphatiques poly hydroxylés saturés ou insaturés;
et leurs mélanges.

De façon préférentielle, le ou les acides carboxyliques hydroxylés sont choisis parmi :
- l'acide 12-hydroxy stéarique ; l'acide α-hydroxy octadécanoïque ; l'acide hydroxy 14-eicosènoïque
- l'acide leucinique ou l'acide 2-éthyl 3-hydroxy caprylique ;
- l'acide ricinoléïque ;
- l'acide 3-hydroxy 4-hexanoïque ou l'acide oxynervonique ;
- l'acide 16-hydroxy 6-hexadécènoïque ;
- l'acide 9, 10-dihydroxy octadécanoïque, l'acide 9, 12-dihydroxy octadécanoïque, l'acide aleuritique, l'acide 9, 10, 12-trihydroxy octadécanoïque, l'acide hexahydroxy octadécanoïque ou l'acide octahydroxy octadécanoïque ;
et leurs mélanges.

Le ou les acides carboxyliques hydroxylés sont de préférence choisis parmi les acides gras insaturés comprenant 16 à 20 atomes de carbone, de préférence 18 atomes de carbone.

Le triglycéride est de préférence le triglycéride de l'acide ricinoléïque. Ce triglycéride se trouve en grande quantité à l'état naturel dans l'huile de ricin.

Le triglycéride d'acide(s) carboxylique(s) hydroxylé(s) est avantageusement choisi parmi les triglycérides d'acides hydroxylés tels que lesdits acides hydroxylés comprennent de 6 à 40 atomes de carbone, de préférence de 10 à 34 atomes de carbone et mieux de 12 à 28 atomes de carbone, de préférence de 16 à 20 atomes de carbone, de préférence 18 atomes de carbone.

L'acide monocarboxylique aliphatique peut être un acide gras aliphatique saturé ou insaturé, comme l'acide isostéarique.

L'acide dicarboxylique aliphatique comprend de préférence de 3 à 10 atomes de carbones, de préférence de 3 à 6 atomes de carbone, de préférence de 3 à 5 atomes de carbone. Selon un mode de mise en oeuvre, l'acide dicarboxylique aliphatique correspond à la formule HOOC-(CH₂)ₙ-COOH telle que n=1 à 4.

L'acide dicarboxylique aliphatique est de préférence l'acide succinique, correspondant à la formule précédente dans laquelle n = 2.

Selon un mode de mise en oeuvre préféré, le polyester répond à la formule

T₂O-(OC-D-CO-O-T₁-O)x-OC-D-CO-OT₂ (I)

dans laquelle
T₂-OH représente un triglycéride d'acide(s) carboxylique(s) hydroxylé(s), ledit triglycéride ayant été estérifié par deux molécules d'un acide monocarboxylique aliphatique, et ledit triglycéride comprenant une seule fonction hydroxyle libre ;
OH-T₁-OH représente un triglycéride d'acide(s) carboxylique(s) hydroxylé(s), ledit triglycéride ayant été estérifié par une molécule d'acide monocarboxylique aliphatique, et ledit triglycéride comprenant deux fonctions hydroxyles libres ;
HOOC-D-COOH représente ledit acide dicarboxylique et
x est compris entre 1 et 50, de préférence entre 1 et 10, de préférence encore entre 2 et 6.

x peut être égal à 3, 4, 5, 6, 7, 8, 9 ou 10.

Le polyester est avantageusement un des polyesters décrits dans le brevet US 6 342 527 dont le contenu est inclus par référence dans la présente demande. Le polyester en question répond à la formule (1) ci-dessus dans laquelle
T₂O-représente 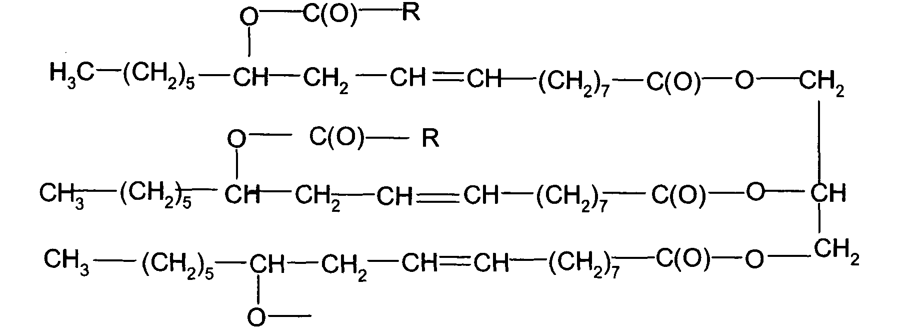 - OT₁O- représente

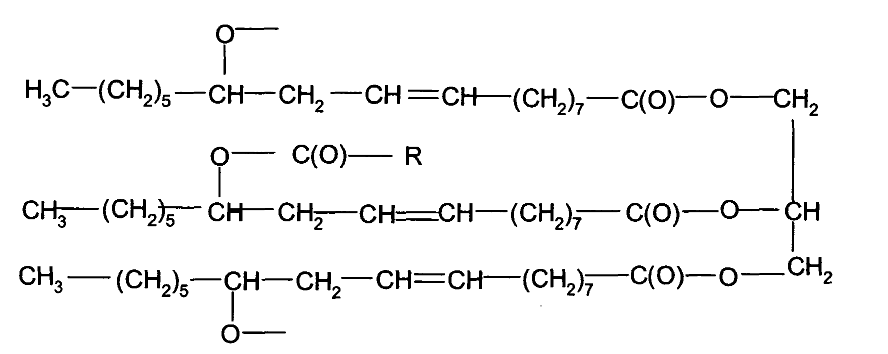

Dans ces formules, R représente un groupe alkyle ou alkylène comprenant 5 à 33 atomes de carbone,

R représente de préférence un alkyle ayant 7 à 17 atomes de carbones ou un alkylène ayant de 11 à 21 atomes de carbones.

Le polyester de la composition de l'invention peut représenter de 0,1 à 99,9 % du poids total de la composition, de préférence de 1 à 99 %, mieux de 1 à 80 %, encore mieux de 10 à 40%, encore mieux de 15 à 25% encore mieux de 20 à 25 %, et de façon générale, être présent en une quantité suffisante pour conférer à la composition des propriétés de brillance, de stabilité, de tenue de la couleur dans le temps, de tenue de la brillance, de confort, de non migration et/ou de netteté des contours du dépôt.

### Pâteux

Par "pâteux" au sens de la présente invention, on entend un composé gras lipophile à changement d'état solide/liquide réversible, présentant à l'état solide une organisation cristalline anisotrope, et comportant à la température de 23°C une fraction liquide et une fraction solide.

Par composé pâteux au sens de l'invention, on entend un composé ayant une dureté à 20°C allant de 0,001 à 0,5 MPa, de préférence de 0,002 à 0,4 MPa.

La dureté est mesurée selon une méthode de pénétration d'une sonde dans un échantillon de composé et en particulier à l'aide d'un analyseur de texture (par exemple le TA-XT2i de chez Rhéo) équipé d'un cylindre en inox de 2 mm de diamètre. La mesure de dureté est effectuée à 20°C au centre de 5 échantillons. Le cylindre est introduit dans chaque échantillon à une pré-vitesse de 1 mm/s puis à une vitesse de mesure de 0,1 mm/s, la profondeur de pénétration étant de 0,3 mm. La valeur relevée de la dureté est celle du pic maximum.

Ce composé pâteux est en outre, à la température de 23°C, sous la forme d'une fraction liquide et d'une fraction solide. En d'autres termes, la température de fusion commençante du composé pâteux est inférieure à 23°C. La fraction liquide du composé pâteux mesurée à 23°C représente 9 à 97% en poids du composé. Cette fraction liquide à 23°C représente de préférence entre 15 et 85%, de préférence encore entre 40 et 85% en poids.

La fraction liquide en poids du composé pâteux à 23°C est égale au rapport de l'enthalpie de fusion consommée à 23°C sur l'enthalpie de fusion du composé pâteux.

L'enthalpie de fusion du composé pâteux est l'enthalpie consommée par le composé pour passer de l'état solide à l'état liquide. Le composé pâteux est dit à l'état solide lorsque l'intégralité de sa masse est sous forme solide cristalline. Le composé pâteux est dit à l'état liquide lorsque l'intégralité de sa masse est sous forme liquide.

L'enthalpie de fusion du composé pâteux est égale à l'aire sous la courbe du thermogramme obtenu à l'aide d'un calorimètre à balayage différentiel (D. S. C), tel que le calorimètre vendu sous la dénomination MDSC 2920 par la société TA instrument, avec une montée en température de 5 ou 10°C par minute, selon la norme ISO 11357-3:1999. L'enthalpie de fusion du composé pâteux est la quantité d'énergie nécessaire pour faire passer le composé de l'état solide à l'état liquide. Elle est exprimée en J/g.

L'enthalpie de fusion consommée à 23°C est la quantité d'énergie absorbée par l'échantillon pour passer de l'état solide à l'état qu'il présente à 23°C constitué d'une fraction liquide et d'une fraction solide.

La fraction liquide du composé pâteux mesurée à 32°C représente de préférence de 30 à 100% en poids du composé, de préférence de 80 à 100%, de préférence encore de 90 à 100% en poids du composé. Lorsque la fraction liquide du composé pâteux mesurée à 32°C est égale à 100%, la température de la fin de la plage de fusion du composé pâteux est inférieure ou égale à 32°C.

La fraction liquide du composé pâteux mesurée à 32°C est égale au rapport de l'enthalpie de fusion consommée à 32°C sur l'enthalpie de fusion du composé pâteux. L'enthalpie de fusion consommée à 32°C est calculée de la même façon que l'enthalpie de fusion consommée à 23°C.

Le composé pâteux est de préférence choisi parmi les composés synthétiques et les composés d'origine végétale. Un composé pâteux peut être obtenu par synthèse à partir de produits de départ d'origine végétale.

Le composé pâteux est avantageusement choisi parmi
- la lanoline et ses dérivés
- les composés siliconés polymères ou non
- les composés fluorés polymères ou non
- les polymères vinyliques, notamment:
   - les homopolymères d'oléfines
   - les copolymères d'oléfines
   - les homopolymères et copolymères de diènes hydrogénés
   - les oligomères linéaires ou ramifiés, homo ou copolymères de (méth)acrylates d'alkyles ayant de préférence un groupement alkyle en C₈-C₃₀
   - les oligomères homo et copolymères d'esters vinyliques ayant des groupements alkyles en C₈-C₃₀
   - les oligomères homo et copolymères de vinyléthers ayant des groupements alkyles en C₈-C₃₀,
- les polyéthers liposolubles résultant de la polyéthérification entre un ou plusieurs diols en C2-C100, de préférence en C2-C50,
- les esters,
et leurs mélanges.

Le composé pâteux est de préférence polymère, notamment hydrocarboné.

Un composé pâteux siliconé et fluoré préféré est le polyméthyl trifluoropropryl méthylalkyl diméthylsiloxane, fabriqué sous la dénomination X22-1088 par SHIN ETSU.

Lorsque le composé pâteux est un polymère siliconé et/ou fluoré, la composition comprend avantageusement un agent compatibilisant tel que les esters à courte chaîne comme le néopentanoate d'isodécyle.

Parmi les polyéthers liposolubles, on préfère en particulier les copolymères d'éthylène-oxyde et/ou de propylène-oxyde avec des alkylènes-oxydes à longue chaîne en C6-C30, de préférence encore tels que le rapport pondéral de l'éthylène-oxyde et/ou de propylène-oxyde avec alkylènes-oxydes dans le copolymère est de 5:95 à 70:30. Dans cette famille, on citera notamment les copolymères tels que les alkylènes-oxydes à longue chaîne sont disposés en blocs ayant un poids moléculaire moyen de 1.000 à 10.000, par exemple un copolymère bloc de polyoxyethylène/polydodécyle glycol tel que les éthers de dodécanediol (22 mol) et de polyéthylène glycol (45 OE) commercialisés sous la marque ELFACOS ST9 par Akzo Nobel.

Parmi les esters, on préfère notamment
- les esters d'un glycérol oligomère, notamment les esters de diglycérol, en particulier les condensats d'acide adipique et de glycérol, pour lesquels une partie des groupes hydroxyles des glycérols ont réagi avec un mélange d'acides gras tels que l'acide stéarique, l'acide caprique, l'acide stéarique et l'acide isostéarique et l'acide 12-hydroxystéarique, à l'image notamment de ceux commercialisé sous la marque Softisan 649 par la société Sasol
- le propionate d'arachidyle commercialisé sous la marque Waxenol 801 par Alzo,
- les esters de phytostérol,
- les triglycérides d'acides gras et leurs dérivés
- les esters de pentaérythritol
- les polyesters non réticulés résultant de la polycondensation entre un acide dicarboxylique ou un polyacide carboxylique linéaire ou ramifié en C4-C50 et un diol ou un polyol en C2-C50,
- les esters aliphatiques d'ester résultant de l'estérification d'un ester d'acide hydroxycarboxylique aliphatique par un acide carboxylique aliphatique;

L'acide carboxylique aliphatique comprend de 4 à 30 et de préférence de 8 à 30 atomes de carbone. Il est de préférence choisi parmi l'acide héxanoïque, l'acide heptanoïque, l'acide octanoïque, l'acide éthyl-2 héxanoïque, l'acide nonanoïque, l'acide décanoïque, l'acide undécanoïque, acide dodécanoïque, l'acide tridécanoïque, l'acide tétradécanoïque, l'acide pentadécanoïque, l'acide héxadécanoïque, l'acide héxyldécanoïque, l'acide heptadécanoïque, l'acide octadécanoïque, l'acide isostéarique, l'acide nonadécanoïque, l'acide eicosanoïque, l'acide isoarachidique, l'acide octyldodécanoïque, l'acide henéicosanoïque, l'acide docosanoïque, et leurs mélanges.
L'acide carboxylique aliphatique est de préférence ramifié.
L'ester d'acide hydroxy carboxylique aliphatique est avantageusement issu d'un acide carboxylique aliphatique hydroxylé comportant de 2 à 40 atomes de carbone, de préférence de 10 à 34 atomes de carbone et mieux de 12 à 28 atomes de carbone, et de 1 à 20 groupes hydroxyle, de préférence de 1 à 10 groupes hydroxyle et mieux de 1 à 6 groupes hydroxyle. L'ester d'acide hydroxy carboxylique aliphatique est choisi parmi :
a) les esters partiels ou totaux d'acides monocarboxyliques aliphatiques mono hydroxylés linéaires, saturés ;
b) les esters partiels ou totaux d'acides monocarboxyliques aliphatiques mono hydroxylés insaturés ;
c) les esters partiels ou totaux de polyacides carboxyliques aliphatiques mono hydroxylés saturés ;
d) les esters partiels ou totaux de polyacides carboxyliques aliphatiques poly hydroxylés saturés ;
e) les esters partiels ou totaux de polyols aliphatiques en C₂ à C₁₆ ayant réagi avec un mono ou un poly acide carboxylique aliphatique mono ou poly hydroxylé, et leurs mélanges.

Les esters aliphatiques d'ester sont avantageusement choisis parmi :
- l'ester résultant de la réaction d'estérification de l'huile de ricin hydrogénée avec l'acide isostéarique dans les proportions 1 pour 1 (1/1) ou monoisostéarate d'huile de ricin hydrogénée,
- l'ester résultant de la réaction d'estérification de l'huile de ricin hydrogénée avec l'acide isostéarique dans les proportions 1 pour 2 (1/2) ou le diisostéarate d'huile de ricin hydrogénée,
- l'ester résultant de la réaction d'estérification de l'huile de ricin hydrogénée avec l'acide isostéarique dans les proportions 1 pour 3 (1/3) ou triisostéarate d'huile de ricin hydrogénée,
- et leurs mélanges.

Parmi les composés pâteux d'origine végétale, on choisira de préférence un mélange de stérols de soja et de pentaérythritol oxyéthyléné (5OE oxypropyléné (5 OP), commercialisé sous la référence Lanolide par la société VEVY.

Le composé pâteux représente de préférence 1 à 99%, mieux 1 à 60%, mieux 2 à 30% et mieux encore 5 à 15% en poids de la composition.

La composition est avantageusement exempte de lanoline ou de l'un de ses dérivés.

### Formes de la composition

La composition de l'invention peut se présenter sous la forme de composition solide, compactée ou coulée en stick ou en coupelle, pâteuse ou liquide. Avantageusement, elle se présente sous forme solide, à savoir sous forme dure (ne s'écoulant pas sous son propre poids) notamment coulée ou compactée, par exemple en stick ou en coupelle.

Elle peut se présenter sous forme de pâte, de solide ou de crème. Elle peut être une émulsion huile-dans-eau ou eau-dans-huile, un gel anhydre, solide ou souple ou encore sous forme de poudre libre ou compactée et même sous forme biphasique. De préférence, elle se présente sous forme de composition à phase continue huileuse et notamment anhydre ; dans ce cas, elle peut contenir une phase aqueuse à un taux inférieur à 10 % et mieux inférieur à 5% en poids.

### Colorants

Avantageusement, la composition de l'invention peut comprendre, en outre, au moins une matière colorante qui peut être choisie parmi les colorants solubles ou dispersibles dans la composition, les pigments, les nacres et leurs mélanges. Les colorants sont de préférence des colorants liposolubles, bien que les colorants hydrosolubles puissent être utilisés. Cette matière colorante peut représenter de 0,001 à 98 %, de préférence de 0,5 à 85% et mieux de 1 à 60 % du poids total de la composition.

Pour une composition sous forme de pâte ou coulée telle que les rouges à lèvres ou les produits de maquillage du corps, on utilise en général de 0,5 à 50% de matière colorante, de préférence de 2 à 40 % et mieux de 5 à 30%, par rapport au poids total de la composition.

Les colorants liposolubles sont par exemple le rouge Soudan, le D & C Red 17, le D & C Green 6, le β-carotène, l'huile de soja, le brun Soudan, le D & C Yellow 11, le D & C Violet 2, le D & C orange 5, le jaune quinoléine, le rocou. Ils peuvent représenter de 0 à 20 % du poids de la composition et mieux de 0,1 à 6 %. Les colorants hydrosolubles sont notamment le jus de betterave, le bleu de méthylène et peuvent représenter de 0,1 à 6 % en poids de la composition (si présents).

De préférence, la composition de l'invention, comprend une phase particulaire avantageusement colorée pouvant représenter de 0,001 à 50 % du poids total de la composition, de préférence de 0,01 à 40 % et mieux de 0,05 à 30 %, et qui peut comprendre des pigments et/ou des nacres et/ou des charges habituellement utilisés dans les compositions cosmétiques.

Par pigments, il faut comprendre des particules blanches ou colorées, minérales ou organiques, insolubles dans la phase grasse liquide, destinées à colorer et/ou opacifier la composition. Par charges, il faut comprendre des particules incolores ou blanches, minérales ou de synthèse, lamellaires ou non lamellaires. Par nacres, il faut comprendre des particules irisées, notamment produites par certains mollusques dans leur coquille ou bien synthétisées. Ces charges et nacres servent notamment à modifier la texture de la composition.

Les pigments peuvent être présents dans la composition à raison de 0,05 à 30 % (si présents) du poids de la composition finale, et de préférence à raison de 2 à 20 %. Comme pigments minéraux utilisables dans l'invention, on peut citer les oxydes de titane, de zirconium ou de cérium ainsi que les oxydes de zinc, de fer ou de chrome et le bleu ferrique. Parmi les pigments organiques utilisables dans l'invention, on peut citer le noir de carbone, et les laques de baryum, strontium, calcium (D & C Red N°7), aluminium.

Les nacres peuvent être présentes dans la composition à raison de 0,001 à 20 % (si présentes) du poids total de la composition, de préférence à un taux de l'ordre de 1 à 15 %. Parmi les nacres utilisables dans l'invention, on peut citer le mica recouvert d'oxyde de titane, d'oxyde de fer, de pigment naturel ou d'oxychlorure de bismuth tel que le mica titane coloré, les pigments goniochromatiques et par exemple les pigments multicouches interférentiels.

Les charges peuvent être présentes à raison de 0,001 à 35 % (si présentes) du poids total de la composition, de préférence 0,5 à 15 %. On peut notamment citer le talc, le mica, le kaolin, les poudres de Nylon® (Orgasol notamment) et de polyéthylène, les poudres de polytétrafluoroéthylène (Téflon®), l'amidon, le nitrure de bore, des microsphères de copolymères telles que l'Expancel® (Nobel Industrie), le Polytrap® (Dow Corning), le Polypore@ L 200 (Chemdal Corporation) et les microbilles de résine de silicone (Tospearl ® de Toshiba, par exemple), la sillice.

La composition selon l'invention peut contenir au moins un composé non aqueux additionnel choisi parmi les huiles, les cires et leurs mélanges.

### Cires

En particulier, la composition peut contenir au moins une cire.

Par "cire" au sens de la présente invention, on entend un composé gras lipophile, solide à température ambiante (25°C), à changement d'état solide/liquide réversible, ayant une température de fusion supérieure à 30°C pouvant aller jusqu'à 200° C, une dureté supérieure à 0,5 MPa, et présentant à l'état solide une organisation cristalline anisotrope. En portant la cire à sa température de fusion, il est possible de la rendre miscible aux huiles et de former un mélange homogène microscopiquement, mais en ramenant la température du mélange à la température ambiante, on obtient une recristallisation de la cire dans les huiles du mélange.

La dureté peut être mesurée par la méthode dite "du fil à couper le beurre", qui consiste à couper un bâton de rouge à lèvres de 12,7 mm et à mesurer la dureté à 20°C, au moyen d'un dynamomètre DFGHS 2 de la société Indelco-Chatillon se déplaçant à une vitesse de 100 mm/minute. Elle est exprimée comme la force de cisaillement (exprimée en gramme) nécessaire pour couper un stick dans ces conditions. Selon cette méthode la dureté d'une composition en stick selon l'invention va notamment de 50 à 300 g, de préférence de 100 à 250 g et par exemple de 150 à 230 g.

Les cires utilisables dans l'invention sont des composés solides à température ambiante, destinés à structurer la composition en particulier sous forme de stick; elles peuvent être hydrocarbonées, fluorées et/ou siliconées et être d'origine végétale, minérale, animale et/ou synthétique. En particulier, elles présentent une température de fusion supérieure à 40°C et mieux supérieure à 45°C.

Comme cire utilisable dans l'invention, on peut citer celles généralement utilisées dans le domaine cosmétique : elles sont notamment d'origine naturelle comme la cire d'abeilles, la cire de Carnauba, de Candelilla, d'Ouricoury, du Japon, de fibres de liège ou de canne à sucre, de riz, de Montan, la paraffine, les cires de lignite ou microcristalline, la cérésine ou l'ozokérite, les huiles hydrogénées comme l'huile de jojoba ; les cires synthétiques comme les cires de polyéthylène issues de la polymérisation ou copolymérisation de l'éthylène de masse moléculaire en poids comprise entre 400 et 800 et leurs mélanges, les cires de Fischer-Tropsch ou encore des esters d'acides gras comme l'octacosanyl stéarate, les glycérides concrets à 40°C et mieux à 45°C, les cires de silicones comme les alkyl- ou alkoxydiméthicones ayant une chaîne alkyle ou alcoxy de 10 à 45 atomes de carbone, les esters de poly(di)méthylsiloxane solide à 40°C dont la chaîne ester comporte au moins 10 atomes de carbone ; et leurs mélanges.

La nature et la quantité des cires sont fonction des propriétés mécaniques et des textures recherchées. A titre indicatif, la cire peut représenter de 0,01 à 50 %, de préférence de 2 à 40 %, et mieux de 5 à 30 % du poids total de la composition.

### Huiles

Par « huile » on entend un corps gras liquide à température ambiante et pression atmosphérique.

Les huiles peuvent être des huiles hydrocarbonées et/ou siliconées et/ou fluorées. Ces huiles peuvent être d'origine animale, végétale, minérale ou synthétique.

"Par huile hydrocarbonée", on entend une huile comportant principalement des atomes de carbone et d'hydrogène et éventuellement une ou plusieurs fonctions choisies parmi les fonctions hydroxyle, ester, éther, carboxylique. A titre d'exemple d'huile utilisable dans l'invention, on peut citer :
- les huiles hydrocarbonées d'origine animale telles que le perhydrosqualène ;
- les huiles hydrocarbonées végétales telles que les triglycérides liquides d'acides gras de 4 à 24 atomes de carbone comme les triglycérides des acides heptanoïque ou octanoïque ou encore les huiles de tournesol, de maïs, de soja, de courge, de pépins de raisin, de sésame, de noisette, d'abricot, de macadamia, de ricin, d'avocat, les triglycérides des acides caprylique/caprique comme ceux vendus par la société Stearineries Dubois ou ceux vendus sous les dénominations Miglyol 810, 812 et 818 par la société Dynamit Nobel, l'huile de jojoba, de beurre de karité ;
- les hydrocarbures linéaires ou ramifiés, d'origine minérale ou synthétique tels que les huiles de paraffine et leurs dérivés, la vaseline, les polydécènes, le polyisobutène hydrogéné tel que le Parleam ;
- les esters et les éthers de synthèse notamment d'acides gras comme les huiles de formule R₁COOR₂ dans laquelle R₁ représente le reste d'un acide gras supérieur comportant de 1 à 40 atomes de carbone et R₂ représente une chaîne hydrocarbonée contenant de 1 à 40 atomes de carbone avec R₁ + R₂ ≥ 10 comme par exemple l'huile de Purcellin, l'isononanoate d'isononyle, le myristate d'isopropyle, le palmitate d'éthyl 2-hexyle, le stéarate d'octyl 2-dodécyle, l'érucate d'octyl 2-dodécyle, l'isostéarate d'isostéaryle ; les esters hydroxylés comme l'isostéaryl lactate, l'octyl hydroxy stéarate, l'hydroxy stéarate d'octyl dodécyle, le diisostéaryl malate, le citrate de triisocétyle, des heptanoates, octanoates, décanoates d'alcools gras ; des esters de polyol comme le dioctanoate de propylène glycol, le diheptanoate de néopentyl glycol, le diisononanoate de diéthylène glycol ; et les esters du pentaérythritol comme le tétra-isostéarate de pentaérythrytyle ;
- des alcools gras ayant de 12 à 26 atomes de carbone comme l'octyl dodécanol, le 2-butyloctanol, le 2-hexyl décanol, le 2-undécyl pentadécanol, l'alcool oléique ;
- les huiles fluorées éventuellement partiellement hydrocarbonées et/ou siliconées ;
- les huiles siliconées comme les polydiméthylsiloxanes (PDMS) volatiles ou non, linéaires ou cycliques ; les polydiméthylsiloxanes comportant des groupements alkyle, alcoxy ou phényle, pendant ou en bout de chaîne siliconée, groupements ayant de 2 à 24 atomes de carbone ; les silicones phénylées comme les phényl triméthicones, les phényl diméthicones, les phényl triméthylsiloxy diphényl siloxanes, les diphényl diméthicones, les diphényl méthyldiphényl trisiloxanes, les 2-phényl éthyl triméthyl-siloxysilicates,
- leurs mélanges.

Les huiles additionnelles peuvent représenter de 0 à 90 % du poids total de la composition, de préférence de 0,05 à 60 % et mieux de 10 à 55 %.

### Additifs

La composition de l'invention peut comprendre, en outre, tout additif usuellement utilisé dans le domaine concerné, tel que l'eau, des antioxydants, des conservateurs, des neutralisants, des gélifiants lipophiles ou de corps gras liquides, des gélifiants de phase aqueuse, des dispersants, des actifs cosmétiques ou dermatologiques. Ces additifs, à l'exception de l'eau qui peut représenter de 0 à 70 % et par exemple de 1 à 50 %,et mieux ce 1 à 10 % du poids total de la composition, peuvent être présents dans la composition à raison de 0 à 20% du poids total de la composition et mieux de 0 à 10%.

La composition selon l'invention peut se présenter sous la forme d'une composition, colorée ou non, sous forme d'une composition de protection solaire ou de démaquillage ou encore sous forme d'une composition hygiénique. Elle contient notamment des actifs cosmétiques. Elle peut alors être utilisée comme base de soin ou de traitement pour la peau comme les mains ou le visage ou pour les lèvres (baumes à lèvres, protégeant les lèvres du froid et/ou du soleil et/ou du vent) ou encore comme déodorant. Comme actif cosmétique utilisable dans l'invention, on peut citer les vitamines A, E, C, B₃, les provitamines comme le D-panthénol, les actifs apaisants comme l'α-bisabolol, l'aloe vera, l'allantoïne, les extraits de plantes ou les huiles essentielles, les agents protecteurs ou restructurants comme les céramides, les actifs fraîcheur comme le menthol et ses dérivés, les émollients (beurre de cacao, diméthicone), les hydratants (arginine PCA), les actifs antirides, les acides gras essentiels, et leurs mélanges.

La composition de l'invention peut également se présenter sous la forme d'un produit de maquillage de la peau, en particulier du visage comme un fond de teint, un blush, un fard comme un produit de tatouage semi-permanent ou de maquillage des lèvres comme un rouge ou un brillant à lèvres, présentant éventuellement des propriétés de soin ou de traitement, un produit de maquillage des phanères comme par exemple un vernis à ongles, un mascara, un eyeliner, un produit de coloration ou de soin des cheveux.

Bien entendu la composition de l'invention doit être cosmétiquement acceptable, à savoir non toxique et susceptible d'être appliquée sur la peau, les phanères ou les lèvres d'êtres humains.

La composition selon l'invention peut être fabriquée par les procédés connus, généralement utilisés dans le domaine cosmétique ou dermatologique.

L'invention a encore pour objet l'utilisation (i) d'au moins un polyester résultant de l'estérification d'au moins un triglycéride d'acide(s) carboxylique(s) hydroxylé(s) par un acide monocarboxylique aliphatique et par un acide dicarboxylique aliphatique, et (ii) d'au moins un composé pâteux ayant une dureté à 25°C comprise entre 0,001 à 0,5 MPa, de préférence de 0,002 à 0,4 MPa, et dont la fraction liquide à 23°C est comprise entre 9 et 97% en poids, de préférence entre 15 et 85%, de préférence encore entre 40 et 85% en poids, dans une composition pour conférer à un film de ladite composition des propriétés de brillance, de confort, de tenue de la couleur dans le temps, de tenue de la brillance dans le temps lorsqu'il est coloré, de non migration, de bon étalement et/ou de glissant à l'application, pour limiter l'exsudation et/ou pour améliorer la netteté des contours du film, et/ou pour augmenter l'intensité de la couleur du film lorsqu'il est coloré.

L'invention est illustrée plus en détail dans les exemples suivants. Les quantités sont données en pourcentage massique.

| Exemple 1 : Rouge à lèvres | |
|---|---|
| Ester d'huile de ricin, d'acide succinique et d'acide isostéarique (commercialisé sous la référence Zénigloss par Zénitech) | 22 |
| Ethers de dodécanediol (22 mols) et de polyéthylène glycol (45 OE) (commercialisé sous la référence ELFACOS ST9 par Akzo Nobel) | 11 |
| Triglycéride d'acide 2-décyl-tetradécanoïque | 20 |
| Polyisobutène hydrogéné | 10 |
| Malate de di-isostéaryle | 11 |
| Polybutylène | 2,5 |
| Stéarate d'octacosanyle | 5 |
| Mélange de triglycérides d'acides laurique, myristique, palmitique, stearique (50/20/10/10) | 2 |
| Cire de polyéthylène | 5 |
| Hectorite (modifiée par le chlorure de di-stéaryl di-méthyl ammonium) | 3 |
| Pigments | qs |
| Conservateur | qs |
| Parfum | qs |

- La phase huileuse est réalisée en mélangeant le conservateur, toutes les huiles ainsi que le pâteux (éthers de dodécanediol (22 mols) et de polyéthylène glycol(45 OE)).
- Puis l'hectorite est broyée dans la phase huileuse à la tricylindre.
- Les pigments sont ensuite broyés dans le mélange hectorite et phase huileuse.
- On ajoute le mélange obtenu dans un poêlon avec les cires et on chauffe à 105°C pendant deux heures en homogénéisant à l'aide d'un appareil Raynerie.
- On ajoute enfin le parfum, on homogénéise 5 minutes puis on coule le mélange dans un moule à 42°C qui est placé à -20°C pendant 30 minutes. Puis on procède au démoulage des sticks.

La formule ci-dessus présente une bonne tenue de la brillance à 1 heure et ne migre pas à 1 heure. Elle possède en outre de bonnes propriétés en terme d'application (glissant), de confort, de brillance (à l'application et dans le temps) et de tenue de la couleur après épreuve.

| Exemple 2: Rouge à lèvres | |
|---|---|
| Ester d'huile de ricin, d'acide succinique et d'acide isostéarique (commercialisé sous la référence Zénigioss par Zénitech) | 24 |
| Bis-diglycéryl polyacyladipate-2 (commercialisé sous la référence Softisan 649 par Sasol) | 12 |
| Huilde sésame | 36,1 |
| Cire d'abeille polyglycérolée (Cera Bellina commercialisée par | |
| Koster Keunen) | 4,2 |
| Cire microcristalline | 10,5 |
| Hectorite (modifiée par le chlorure de di-stéaryl di-méthyl ammonium) | 0,6 |
| Pigments | qs |
| Conservateur | qs |
| Parfum | qs |

La formule ci-dessus possède de bonnes propriétés en terme d'application (glissant), de confort, de brillance (à l'application et dans le temps).

## Revendications

1. Composition cosmétique contenant i) au moins un polyester résultant de l'estérification d'au moins un triglycéride d'acide(s) carboxylique(s) hydroxylé(s) a) par un acide monocarboxylique aliphatique et b) par un acide dicarboxylique aliphatique, et ii) un composé pâteux.

2. Composition selon la revendication 1, caractérisée en ce ledit polyester est obtenu par
a) l'estérification par un acide monocarboxylique aliphatique d'une partie des fonctions hydroxyles d'un triglycéride d'acide(s) carboxylique(s) hydroxylé(s), puis par
b) l'estérification par un acide dicarboxylique des fonctions hydroxyles restantes dudit triglycéride d'acide(s) carboxylique(s) hydroxylé(s) estérifié par ledit acide monocarboxylique aliphatique.

3. Composition selon l'une des revendications précédentes, **caractérisée en ce que** le triglycéride d'acide(s) carboxylique(s) hydroxylé(s) est choisi parmi les triglycérides d'acide(s) carboxylique(s) hydroxylé(s) tels que ledit ou lesdits acides hydroxylés comprennent de 6 à 40 atomes de carbone, de préférence de 10 à 34 atomes de carbone et mieux de 12 à 28 atomes de carbone, de préférence de 16 à 20 atomes de carbone, de préférence 18 atomes de carbone.

4. Composition selon l'une quelconque des revendications précédentes, **caractérisée en ce que** le triglycéride d'acide(s) carboxylique(s) hydroxylé(s) est choisi parmi les triglycéride d'acide(s) carboxylique(s) hydroxylé(s) tels que:
i) les acides monocarboxyliques aliphatiques mono hydroxylés linéaires saturés de formule :
(1) 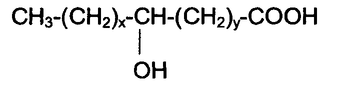 avec 3 ≤ x + y ≤ 37
ou (2) HO-CH₂-(CH₂)ₓ-COOH avec 4 ≤ x ≤ 38 ;
ii) les acides monocarboxyliques aliphatiques mono hydroxylés ramifiés saturés de formule :
(3) 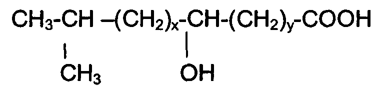 avec 1 ≤ x + y ≤ 35
Ou (3') l'acide 2-éthyl 3-hydroxy caprylique de formule : 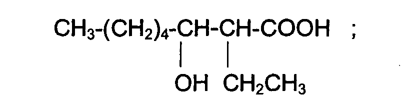
iii) les acides monocarboxyliques aliphatiques mono hydroxylés insaturés de formule :
(4) 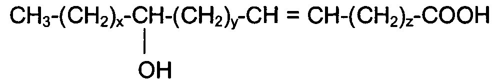 avec 1 ≤ x + y + z ≤ 35
ou (5) 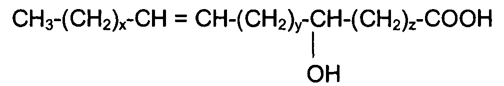 avec 1 ≤ x + y + z ≤ 35
ou (6) HOCH₂-(CH₂)ₓ-CH = CH-(CH₂)_{y}-COOH avec 2 ≤ x + y ≤ 36 ;
iv) les acides monocarboxyliques aliphatiques poly hydroxylés saturés de formule :
(7) 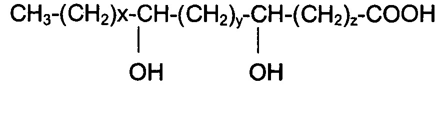 avec 2 ≤ x + y + z ≤ 36; et les acides monocarboxyliques aliphatiques poly hydroxylés insaturés correspondants,
v) les polyacides aliphatiques mono hydroxylés saturés de formule :
(8) 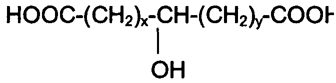 avec 3 ≤ x + y ≤ 37 ; et les polyacides aliphatiques mono hydroxylés insaturés correspondants,
vi) les polyacides aliphatiques poly hydroxylés saturés ou insaturés;
et leurs mélanges.

5. Composition selon l'une quelconque des revendications précédentes, **caractérisée en ce que** le triglycéride d'acide(s) carboxylique(s) hydroxylé(s) est choisi parmi les triglycérides d'acide(s) carboxylique(s) hydroxylé(s) tels que:
- l'acide 12-hydroxy stéarique ; l'acide α-hydroxy octadécanoïque; l'acide hydroxy 14-eicosènoïque;
- l'acide leucinique ou l'acide 2-éthyl 3-hydroxy caprylique ;
- l'acide ricinoléïque ;
- l'acide 3-hydroxy 4-hexanoïque ou l'acide oxynervonique ;
- l'acide 16-hydroxy 6-hexadécènoïque ;
- l'acide 9, 10-dihydroxy octadécanoïque, l'acide 9, 12-dihydroxy octadécanoïque, l'acide aleuritique, l'acide 9, 10, 12-trihydroxy octadécanoïque, l'acide hexahydroxy octadécanoïque ou l'acide octahydroxy octadécanoïque ;
et leurs mélanges.

6. Composition selon l'une des revendications précédentes, **caractérisée en ce que** le triglycéride est le triglycéride de l'acide ricinoléïque

7. Composition selon l'une quelconque des revendications précédentes, **caractérisée en ce que** l'estérification dudit triglycéride d'acide(s) carboxylique(s) hydroxylé(s) est effectuée avec un acide monocarboxylique aliphatique comprenant de 6 à 40 atomes de carbones, de préférence de 10 à 34 atomes de carbone et mieux de 12 à 28 atomes de carbone, de préférence de 16 à 20 atomes de carbone, de préférence 18 atomes de carbone.

8. Composition selon la revendication précédente, **caractérisée en ce que** l'acide monocarboxylique aliphatique est un acide gras aliphatique saturé ou insaturé.

9. Composition selon la revendication précédente, **caractérisée en ce que** l'acide gras est l'acide isostéarique.

10. Composition l'une quelconque des revendications précédentes, **caractérisée en ce que** l'acide dicarboxylique aliphatique comprend de 1 à 10 atomes de carbones, de préférence de 1 à 6 atomes de carbone.

11. Composition selon la revendication précédente, **caractérisée en ce que** l'acide dicarboxylique aliphatique correspond à la formule HOOC-(CH₂)ₙ-COOH telle que n=1 à 4.

12. Composition selon la revendication précédente, **caractérisée en ce que** l'acide dicarboxylique aliphatique est l'acide succinique, tel que n = 2.

13. Composition selon la revendication 1, **caractérisée en ce que** le polyester répond à la formule
T₂O-(OC-D-CO-O-T₁-O)x-OC-D-CO-OT₂ (I)
dans laquelle T₂-O- provient du composé T₂-OH qui représente un triglycéride d'acide(s) carboxylique(s) hydroxylé(s) comprenant une seule fonction hydroxyle libre.
- O-T₁-O- provient du composé HO-T₁-OH qui représente un triglycéride d'acide(s) carboxylique(s) hydroxylé(s) comprenant deux fonctions hydroxyles libres.
- OC-D-CO- provient du composé HOOC-D-COOH qui représente ledit acide dicarboxylique, et
x est compris entre 1 et 50, de préférence entre 1 et 10, de préférence encore entre 2 et 6.

14. Composition selon la revendication 13, **caractérisée en ce que** T₂-OH représente un triglycéride d'acide(s) carboxylique(s) hydroxylé(s), ledit tryglycéride étant estérifié par deux molécules d'un acide monocarboxylique aliphatique.

15. Composition selon la revendication 13, **caractérisée en ce que** HO-T₁-OH représente un triglycéride d'acide(s) carboxylique(s) hydroxylé(s), ledit tryglycéride étant estérifié par une molécule d 'un acide monocarboxylique aliphatique.

16. Composition selon la revendication 13, **caractérisée en ce que** le polyester répond à la formule (1) dans laquelle
T₂O représente 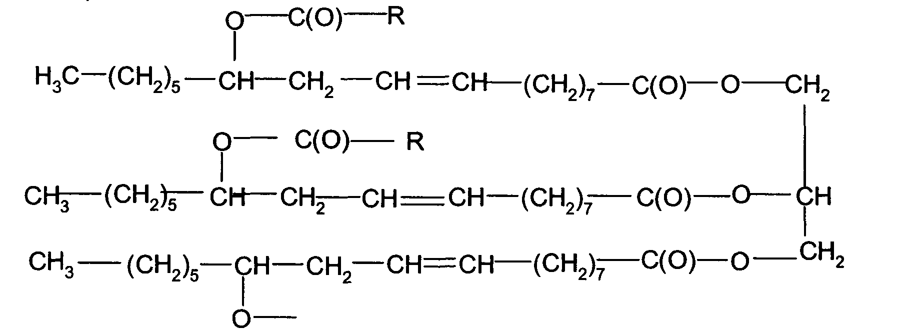 O-T₁-O représente 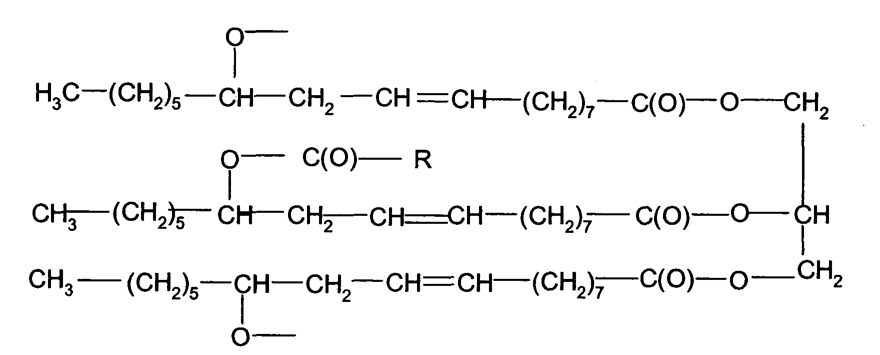 dans lesquelles R représente un groupe alkyle ou alkylène comprenant 5 à 33 atomes de carbone,

17. Composition selon la revendication précédente, **caractérisée en ce que** R représente un alkyle ayant 7 à 17 atomes de carbones.

18. Composition selon la revendication 16, **caractérisée en ce que** R représente un alkylène ayant de 11 à 21 atomes de carbones.

19. Composition selon l'une des revendications précédentes, **caractérisée en ce que** le polyester est liquide à température ambiante.

20. Composition selon l'une des revendications précédentes, **caractérisée en ce que** le polyester présente une viscosité supérieure à 500 cP (50 Pa.s) à 25°C, de préférence allant de 900 à 10 000 cP (90 à 1 000 Pa.s) et mieux de 950 à 5 000 cP (95 à 500 Pa.s) et/ou un indice de réfraction ≥ 1,48.

21. Composition selon l'une des revendications précédentes, **caractérisée en ce que** le polyester est présent en une quantité suffisante pour conférer à la composition des propriétés de non gras, de glissant, de brillance, de stabilité, de tenue de la couleur dans le temps, de tenue de la brillance dans le temps, de confort, de non migration et/ou de netteté des contours du dépôt de ladite composition.

22. Composition selon l'une des revendications précédentes, **caractérisée en ce que** le polyester est présent en une quantité allant de 10 à 40 %, de préférence de 15 à 25 % et mieux de 20 à 25 % du poids total de la composition.

23. Composition selon l'une des revendications précédentes, **caractérisée en ce que** le composé pâteux a une dureté à 20°C comprise entre 0,001 à 0,5 MPa, de préférence de 0,002 à 0,4 MPa.

24. Composition selon l'une des revendications précédentes, **caractérisée en ce que** le composé pâteux a une fraction liquide à 23°C comprise entre 9 et 97% en poids, de préférence entre 15 et 85%, de préférence encore entre 40 et 85% en poids.

25. Composition selon l'une des revendications précédentes, **caractérisée en ce que** le composé pâteux a une fraction liquide à 32°C comprise entre 30 et 100% en poids, de préférence entre 80 et 100%, de préférence encore entre 90 et 100% en poids.

26. Composition selon l'une des revendications précédentes, **caractérisée en ce que** le composé pâteux est choisi parmi
- les composés siliconés polymères ou non,
- les composés fluorés polymères ou non,
- les polymères vinyliques, notamment:
• les homopolymères d'oléfines
• les copolymères d'oléfines
• les homopolymères et copolymères de diènes hydrogénés
• les oligomères linéaires ou ramifiés, homo ou copolymères de (méth)acrylates d'alkyles ayant de préférence un groupement alkyle en C₈-C₃₀
• les oligomères homo et copolymères d'esters vinyliques ayant des groupements alkyles en C₈-C₃₀
• les oligomères homo et copolymères de vinyléthers ayant des groupements alkyles en C₈-C₃₀,
- les polyéthers liposolubles résultant de la polyéthérification entre un ou plusieurs diols en C2-C100, de préférence en C2-C50,
- les esters,
et leurs mélanges.

27. Composition selon l'une des revendications précédentes, **caractérisée en ce que** le composé pâteux est hydrocarboné.

28. Composition selon la revendication 26, **caractérisée en ce que** le composé pâteux est le polyméthyl trifluoropropryl méthylalkyl diméthylsiloxane.

29. Composition selon la revendication 26, **caractérisée en ce que** le polyéther liposoluble est choisi parmi les copolymères d'éthylène-oxyde et/ou de propylène-oxyde avec des alkylènes oxydes à longue chaîne en C6-C30, de préférence encore tels que le rapport pondéral de l'éthylène-oxyde et/ou de propylène-oxyde aux alkylènes-oxydes dans le copolymère est de 5:95 à 70:30.

30. Composition selon la revendication 29, **caractérisée en ce que** le polyéther liposoluble est un copolymère bloc de polyoxyéthylène/polydodécyle glycol.

31. Composition selon la revendication 26, **caractérisée en ce que** les esters sont choisis parmi
- les esters d'un glycérol oligomère, notamment les esters de diglycérol, en particulier les condensats d'acide adipique et de glycérol, pour lesquels une partie des groupes hydroxyles des glycérols ont réagi avec un mélange d'acides gras tels que l'acide stéarique, l'acide caprique, l'acide stéarique et l'acide isostéarique et l'acide 12-hydroxystéarique,
- le propionate d'arachidyle,
- les esters de phytostérol,
- les triglycérides d'acides gras et leurs dérivés,
- les esters de pentaérythritol,
- les polyesters non réticulés résultant de la polycondensation entre un acide dicarboxylique ou un polyacide carboxylique linéaire ou ramifié en C4-C50 et un diol ou un polyol en C2-C50, et
- les esters aliphatiques d'ester résultant de l'estérification d'un ester d'acide hydroxycarboxylique aliphatique par un acide carboxylique aliphatique,
- et leurs mélanges.

32. Composition selon l'une des revendications précédentes, se présentant sous forme de produit de maquillage du corps, d'un rouge ou brillant à lèvres, d'un mascara, d'un vernis à ongles, d'un produit de coloration ou de soin des cheveux, d'un déodorant.

33. Composition selon l'une des revendications précédentes, **caractérisée en ce qu'**elle se présente sous forme coulée ou compactée.

34. Composition selon l'une des revendications précédentes, **caractérisée en ce qu'**elle se présente sous forme d'un rouge à lèvres.

35. Utilisation (i) d'au moins un polyester résultant de l'estérification d'au moins un triglycéride d'acide(s) carboxylique(s) hydroxylé(s) par au moins un acide monocarboxylique aliphatique et par un acide dicarboxylique aliphatique, et (ii) d'au moins un composé pâteux ayant une dureté à 25°C comprise entre 0,001 à 0,5 MPa, de préférence de 0,002 à 0,4 MPa, et dont la fraction liquide à 23°C est comprise entre 9 et 97% en poids, de préférence entre 15 et 85%, de préférence encore entre 40 et 85% en poids.
dans une composition
pour conférer à un film de ladite composition des propriétés de non gras, de brillance, de confort, de tenue de la couleur dans le temps, de tenue de la brillance dans le temps, de non migration, de bon étalement et/ou de glissant à l'application, de confort, de non migration, de netteté des contours dudit film, d'intensité de la couleur, et/ou pour limiter l'exsudation.
